# EUROPÄISCHE PATENTANMELDUNG

(11) **EP 1 271 343 A1**
(43) Veröffentlichungstag der Anmeldung: **02.01.2003**
(21) Anmeldenummer: 01115230.3
(22) Anmeldetag: 22.06.2001
(51) Int. Cl.: G06F 17/30

(54) **Verfahren und Vorrichtung zum automatischen Verarbeiten und Ausgeben von elektronischen Datenmengen**

(71) Anmelder: Kelman Gesellschaft für Geninformation mbH, 12555 Berlin (DE)
(72) Erfinder: Rieger, Peter, 10827 Berlin (DE); Merkel, Dieter, Dr., 12623 Berlin (DE); Kilian, Axel, 12043 Berlin (DE); Wegner, Gunnar, 13156 Berlin (DE); Heymann, Stephan, Dr., 10435 Berlin (DE)
(74) Vertreter: Liesegang, Eva

(57) **Zusammenfassung**

Die Erfindung betrifft ein Verfahren und eine Vorrichtung zum automatischen Verarbeiten und Ausgeben von elektronischen Daten aus einer Datenspeichereinrichtung, wobei die elektronischen Daten eine erste Folge elektronischer Abfragedaten, Zumindest eine zweite Folge elektronischer Abfragedaten und elektronische Testdaten umfassen, wobei die erste Folge elektronischer Abfragedaten eine erste Folge von Objekten elektronisch repräsentiert, die wenigstens eine gemeinsame Eigenschaft aufweisen, die zweite Folge elektronischer Abfragedaten eine zweite Folge von Objekten elektronisch repräsentiert, die wenigstens eine gemeinsame Eigenschaft aufweisen, und wobei die elektronischen Testdaten weinigstens ein Testobjekt elektronisch repräsentieren. Es werden automatisch elektronische Signale zum Ausgeben von Knoten und Kanten erzeugt, wobei Benutzer-Eingaben automatisch berücksichtigt werden. Hierdurch ist eine Möglichkeit geschaffen, aus einer umfangreichen Datensammlung schnell Ergebnisse für verschiedene Anwendungsfälle zu visualisieren.

## Beschreibung

Die Erfindung liegt auf dem Gebiet des automatischen Verarbeitens und Ausgebens umfangreicher Mengen elektronischer Daten, die in einer Datenspeichereinrichtung, insbesondere einer Datenbank, abgelegt sind.

Mit der zunehmenden Leistungsfähigkeit elektronischer Computer- bzw. Rechneranlagen werden auf verschiedenen naturwissenschaftlichen/technischen Gebieten umfangreiche Sammlungen elektronischer Daten geschaffen. Die Ansammlung elektronischer Daten umfaßt Informationen, die es dem Benutzer ermöglichen sollen, Problemstellungen auf verschiedenen naturwissenschaftlichen/technischen Gebieten zu lösen. Hierbei besteht in der Regel das Problem, die gesammelten elektronischen Daten mit Hilfe einer elektronischen Verarbeitungseinrichtung, insbesondere einem Computer, so zu verarbeiten und auf der Basis der Verarbeitung elektrischer Ausgabesignale so zu erzeugen, daß es dem Benutzer ermöglicht ist, die ausgegebenen Signale möglichst schnell und effizient für die Problemlösung zu nutzen.

Das beschriebene Problem besteht beispielsweise auch im Zusammenhang mit gespeicherten, elektronischen Daten, die Informationen über mögliche Bindungspartnerschaften zwischen Proteinen umfassen. Hierbei werden sowohl experimentell als auch mit Hilfe computergestützter Rechnungen gewonnene Aussagen über potentielle Protein-Protein-Wechselwirkungen zur Ausbildung einer oder mehrerer Bindungen zwischen einem Test- bzw. Abfrageprotein und möglichen Wechselwirkungsproteinen getroffen. Die Vorhersageergebnisse umfassen hierbei insbesondere eine Aussage über funktions- und krankheitsrelevante Varianten (Daseinsformen) des Abfrageproteins, die mit potentiellen Wechselwirkungsproteinen wechselwirken. Dieses bedeutet, daß für die Abschnitte der Aminosäurenfolge des Abfrageproteins und die Abschnitte der jeweiligen Aminosäurenfolge der Wechselwirkungsproteine eine Vorhersage über eine möglichen Bindungsbeteiligung getroffen wird. Die große Anzahl von Proteinen, die bei einer Abfrage nach potentiellen Wechselwirkungspartner für das Abfrageprotein in der Regel gefunden wird, führt dazu, daß die Auswertung der hierbei erzeugten elektronischen Daten für den Benutzer sehr zeitaufwendig ist.

Aufgabe der vorliegenden Erfindung ist es deshalb, ein verbessertes Verfahren und eine verbesserte Vorrichtung zum automatischen Verarbeiten und Ausgeben von elektronischen Daten zu schaffen, die ein anwendungsabhängiges Erzeugen und Ausgaben elektronischer Signale und zugehöriger elektronischer Informationen auf der Basis der elektronischen Daten ermöglicht, so daß für einen Benutzer die Auswertemöglichkeiten in Verbindung mit großen Mengen elektronischer Daten erweitert werden.

Nach einem Aspekt der Erfindung ist ein Verfahren zum automatischen Verarbeiten und Ausgeben von elektronischen Daten aus einer Datenspeichereinrichtung geschaffen, wobei die elektronischen Daten eine erste Folge elektronischer Abfragedaten, zumindest eine zweite Folge elektronischer Abfragedaten und elektronische Testdaten umfassen, wobei die erste Folge elektronischer Abfragedaten eine erste Folge von Objekten elektronisch repräsentiert, die wenigstens eine gemeinsame Eigenschaft aufweisen, wobei die zumindest eine zweite Folge elektronischer Abfragedaten eine zweite Folge von Objekten elektronisch repräsentiert, die wenigstens eine gemeinsame Eigenschaft aufweisen, und wobei die elektronischen Testdaten wenigstens ein Testobjekt elektronisch repräsentieren. Das Verfahren umfaßt die folgenden Schritte:
- automatisches Erfassen einer Schlüsselwort-Benutzereingabe;
- Abrufen der ersten Folge elektronischer Abfragedaten über eine Abrufeinrichtung einer elektronischen Verarbeitungseinrichtung, elektronisches Ausbilden eines ersten Knotens, welcher die erste Folge elektronischer Abfragedaten umfaßt, und automatisches Erzeugen erster elektronischer Signale in der elektronischen Verarbeitungseinrichtung zum Ausgeben einer grafischen Repräsentation des ersten Knotens;
- Abrufen der zumindest einen zweiten Folge elektronischer Abfragedaten über die Abrufeinrichtung der elektronischen Verarbeitungseinrichtung, elektronisches Ausbilden zumindest eines zweiten Knotens, welcher die zumindest eine zweite Folge elektronischer Abfragedaten umfaßt, und automatisches Erzeugen zweiter elektronischer Signale zum Ausgeben einer grafischen Repräsentation des zweiten Knotens;
- Abrufen wenigstens eines Teils der elektronischen Testdaten über die Abrufeinrichtung der elektronischen Verarbeitungseinrichtung, elektronisches Ausbilden wenigstens eines weiteren Knotens, welcher den wenigstens einen Teil der elektronischen Testdaten umfaßt, und automatisches Erzeugen weiterer elektronischer Signale zum Ausgeben einer grafischen Repräsentation des wenigstens einen weiteren Knotens;
- elektronisches Ausbilden von Kanten zwischen dem ersten Knoten und/oder dem zweiten Knoten und/oder dem weiteren Knoten in Abhängigkeit von der erfaßten Schlüsselwort-Benutzereingabe und automatisches Erzeugen anderer elektronischer Signale zum Ausgeben einer grafischen Repräsentation der Kanten mit Hilfe der elektronischen Verarbeitungseinrichtung; und
- Übermitteln der ersten elektronischen Signale, der zweiten elektronischen Signale, der weiteren elektronischen Signale und der anderen elektronischen Signale sowie jeweils zugehöriger Informationen zum Unterscheiden des ersten Knotens, des zumindest einen zweiten Knotens, des weiteren Knotens bzw. der Kanten von der elektronischen Verarbeitungseinrichtung an wenigstens eine Ausgabeeinrichtung zum Ausgeben über die wenigstens eine Ausgabeeinrichtung.

Mit Hilfe des neuen Verfahrens ist es dem Benutzer ermöglicht, aus einer großen Anzahl elektronischer Daten, die elektronische Signale und zugehörige Informationen zur Unterscheidung der elektronische Signale umfassen, die für einen Anwendungsfall relevanten elektronischen Daten schnell herauszufiltern und darzustellen. Der Benutzer kann mit Hilfe einer Schlüsselwort-Eingabe das automatische Verarbeiten und Ausgeben der elektronischen Daten für die konkrete Anwendung einstellen und optimieren. Insbesondere in Verbindung mit der Auswertung von umfangreichen elektronischen Datensammlungen, die Informationen über die potentielle Wechselwirkung zwischen Proteinen auf der Basis zugehöriger Geninformation umfassen, ermöglicht das Verfahren eine strukturierte, automatische Aufbereitung und Ausgabe der vorhandenen Daten.

Mit Hilfe von Eingaben kann der Benutzer hierbei das automatische Verarbeiten der elektronischen Daten beeinflussen und verändern, so daß eine schnelle und effiziente Aufbereitung und Visualisierung der elektronischen Daten ermöglicht ist.

Die Erfindung wird anhand von Ausführungsbeispielen unter Bezugnahme auf eine Zeichnung näher erläutert. Hierbei zeigen:
- Figur 1: eine schematische Blockdarstellung einer Anordnung zum automatischen Verarbeiten und Ausgeben elektronischer Daten;
- Figur 2: eine schematische Darstellung von zwei Knoten, die über Kanten jeweils mit einem weiteren Knoten verbunden sind;
- Figur 3: die beiden Knoten nach Figur 2, wobei die beiden Knoten jeweils Unterknoten aufweisen, die wenigsten teilweise mit dem weiteren Knoten über Kanten verbunden sind;
- Figur 4: die beiden Knoten nach Figur 2, wobei Unterknoten über eine Kante verbunden sind;
- Figur 5: eine schematische Darstellung von weiteren Knoten, die über Kanten verbunden sind und Unterknoten umfassen;
- Figur 6: eine schematische Darstellung der Knoten nach Figur 5, wobei in Abhängigkeit von einer Benutzereingabe eine andere Kante gezeigt ist; und
- Figur 7: eine schematische Darstellung von Knoten und Kanten auf einer Kugeloberfläche.

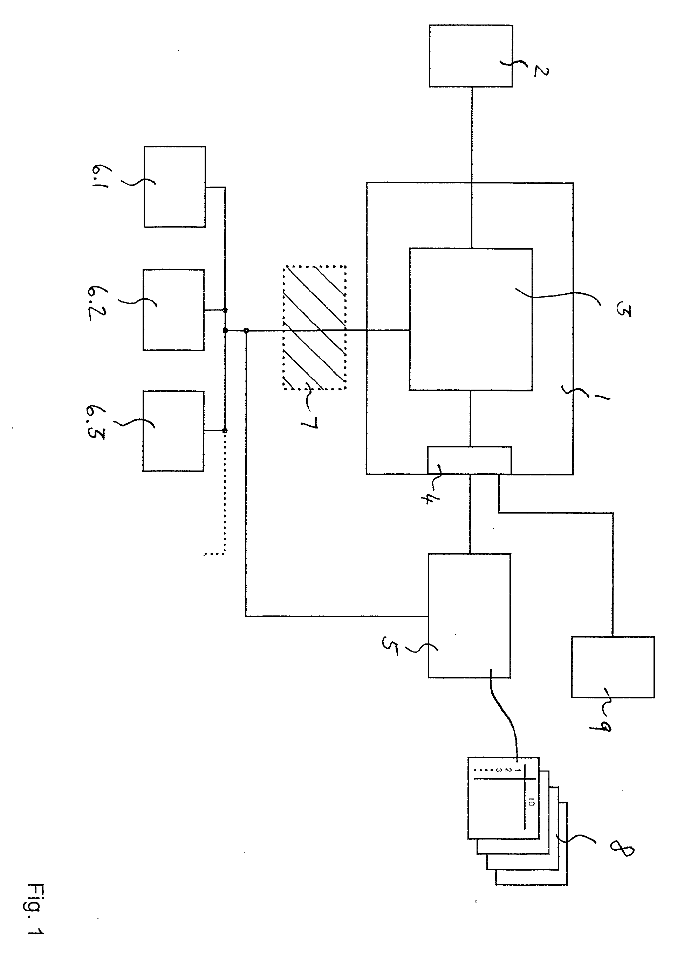

Gemäß Figur 1 ist eine elektronische Verarbeitungseinrichtung 1 mit einer Eingabeinrichtung 2 verbunden. Die elektronische Verarbeitungseinrichtung 1 kann insbesondere ein Computer bzw. eine Rechenanlage sein. Mit Hilfe der Eingabeeinrichtung 2 kann ein Benutzer Eingabebefehle eingeben. Die mit Hilfe der Eingabebefehle erzeugten elektronischen Signale dienen der Steuerung der elektronischen Verarbeitungseinrichtung 1, wobei vorzugsweise eine interaktive Steuerung ausgebildet ist.

Zum Verarbeiten elektronischer Daten, d.h. elektronischer Signale und zugehöriger Informationen zur Unterscheidung der elektronischen Signale, weist die elektronische Verarbeitungseinrichtung 1 eine Prozessoreinheit 3 auf. Mit Hilfe der Prozessoreinheit 3 können über eine Schnittstellen- bzw. Abrufeinrichtung 4 elektronische Daten aus einer Speichereinrichtung 5 abgerufen bzw. an die Speichereinrichtung 5 übertragen werden. Die Speichereinrichtung 5 ist in Figur 1 nicht von der elektronischen Verarbeitungseinrichtung 1 umfaßt, kann jedoch als Teil der elektronischen Verarbeitungseinrichtung 1 ausgebildet sein (nicht dargestellt). Bei der Speichereinrichtung 5 kann es sich um eine Festplattenspeicher oder einen anderen geeigneten Speicher zum abrufbaren Speichern elektronischer Daten handeln. Die Speichereinrichtung 5 kann von einer Datenbankeinrichtung umfaßt sein.

Elektronische Signale, die mit Hilfe der elektronischen Daten erzeugt werden, die über die Schnittstelleneinrichtung 4 von der Speichereinrichtung 5 abgerufen und mit Hilfe der Prozessoreinheit 3 verarbeitet werden, können über eine oder mehrere Ausgabeeinrichtungen 6.1, 6.2, ... ausgegeben werden. Bei den Ausgabeeinrichtungen 6.1, 6.2, ... kann es sich beispielsweise um eine Bildschirmeinrichtung, einen Drucker, einen Plotter oder dergleichen handeln. Es kann auch vorgesehen sein, daß die mehreren Ausgabeeinrichtungen 6.1, 6.2, ... eine weitere Speichereinrichtung umfassen, in welcher die mit Hilfe der Prozessoreinheit 3 erzeugten elektronischen Signale und zugehörige Informationen zur Unterscheidung der Signale als elektronische Daten gespeichert werden.

Gemäß Figur 1 kann auch eine Ausführungsform vorgesehen sein, bei der die mit Hilfe der Prozessoreinheit 3 erzeugten elektronischen Signale sowie zugehörige Informationen zur Charakterisierung der elektronischen Signale in der Speichereinrichtung 5 abgelegt werden.

Eine zweckmäßige Ausgestaltung sieht vor, daß die mittels der Prozessoreinheit 3 erzeugten elektronischen Daten von den mehreren Ausgabeeinrichtungen 6.1, 6.2, ... über einen Internetabruf heruntergeladen und anschließend ausgegeben werden. In Figur 1 ist dieses schematisch mit Hilfe eines Blocks 7 dargestellt.

Zur Erläuterung des Zusammenwirkens der Komponenten, die in Figur 1 schematisch als Blockelemente dargestellt sind, wird im Folgenden angenommen, daß in der Speichereinrichtung 5 elektronische Daten gespeichert sind, die elektronische Signale und Informationen betreffend eine Liste 8 von möglichen Wechselwirkungspartnern einer Test- bzw. Abfragesequenz umfassen. Bei der Abfragesequenz handelt es sich im vorliegenden Beispiel um eine konkrete Daseinsform eines Test- bzw. Abfrageproteins, welche mit Hilfe eines Datenbankschlüssels gespeichert wird. Mit Hilfe des Datenbankschlüssels kann auf die für das Test- bzw. Abfrageprotein gespeicherten Informationen, einschließlich der spezifische Aminosäuresequenz, automatisch zugegriffen werden.

Mit Hilfe eines experimentellen und/oder eines computer-gestützten Verfahrens wurden Proteine aufgefunden, die potentielle Wechselwirkungspartner für das Abfrageprotein darstellen. Die potentiellen Wechselwirkungspartner sind in der Liste 8 elektronisch gespeichert. Auch die potentiellen Wechselwirkungspartner werden mit Hilfe eines jeweiligen Datenbankschlüssels repräsentiert.

Über die Schnittstelleneinrichtung 4 werden die elektronischen Daten 8 aus der Speichereinrichtung 5 durch die Prozessoreinheit 3 abgerufen. Hierbei handelt es sich in der Regel um elektronische Daten, die eine große Anzahl (> 1.000) von potentiellen Wechselwirkungspartner beschreiben. Um die Anzahl der Wechselwirkungspartner einzugrenzen, werden von der Prozessoreinheit 3 über die Schnittstelleneinrichtung 4 weitere elektronisch gespeicherte Informationen, insbesondere aus einer oder mehreren externen Datenbanken 9 automatisch einbezogen. Nach der Verarbeitung der aus der Speichereinrichtung 5 und den externen Datenbanken 9 abgerufenen elektronischen Daten werden mit Hilfe der Prozessoreinheit 3 elektronische Signale automatisch erzeugt, die dann an die Ausgabeeinrichtungen 6.1, 6.2, ... übergeben werden, um in den mehreren Ausgabeeinrichtungen 6.1, 6.2, ... eine Ausgabe automatisch zu erzeugen. Hierbei kann es sich beispielsweise um eine Bildschirmausgabe oder einen Ausdruck mit Hilfe eines Druckers oder eines Plotters handeln.

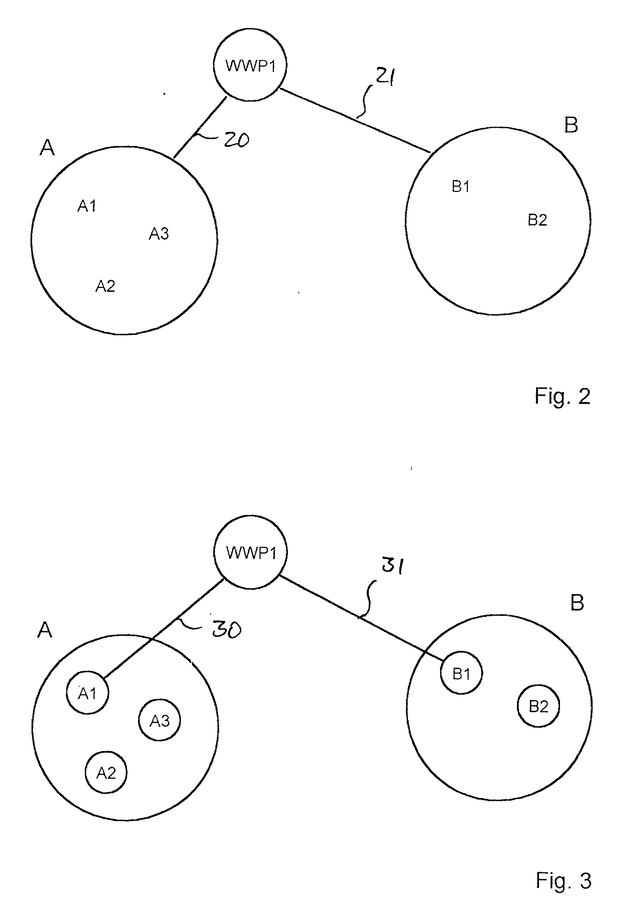

Die Prozessoreinheit 3 erzeugt auf der Basis der von der Datenspeichereinrichtung 5 abgerufenen elektronischen Daten 8 und unter automatischer Berücksichtigung der vom Benutzer über die Eingabeeinrichtung 2 eingegebenen Benutzerbefehle, sofern solche erfaßt wurden, elektronische Signale zum Ausgeben von Knoten und Kanten. In Figur 2 ist eine Ausgabe auf der Basis der erzeugten, elektronischen Signale für die Knoten schematisch dargestellt. Zwei Knoten A, B umfassen Elemente A1, A2, A3 bzw. B1, B2. Die Elemente A1, A2, A3 und B1, B2 repräsentieren jeweils eine Daseinsform eines Proteins, wobei die Elemente A1, A2, A3 Daseinsformen eines Proteins A und die Elemente B1, B2 Daseinsformen eines Proteins B repräsentieren. Verschiedene Daseinsformen können beispielsweise verschiedenen Mutanten eines Gens entsprechen. Die Elemente A1, A2, A3 bzw. B1, B2 des Knotens A bzw. B weisen somit wenigstens eine gemeinsame Eigenschaft auf, derart, daß sie einem jeweiligen Protein zuordbar sind.

Zwischen dem Knoten A und einem weiteren Knoten, der mit WWP1 bezeichnet ist, und zwischen dem Knoten B und dem weiteren Knoten WWP1 ist in Figur 2 eine jeweilige Kante 20 bzw. 21 dargestellt. Die Kanten 20, 21 bringen zum Ausdruck, daß das mit Hilfe des Knoten WWP1 repräsentierte Protein mit wenigstens einer Daseinsform aus dem Knoten A bzw. wenigstens einer Daseinsform aus dem Knoten B potentiell wechselwirkt. Mittels der Prozessoreinrichtung 5 werden auf der Basis der aus der Speichereinrichtung 5 abgerufenen, elektronischen Daten elektronische Signale automatisch so verarbeitet, daß die Knoten und die Kanten gemäß den von den elektronischen Daten umfaßten Informationen hinsichtlich der potentiellen Wechselwirkung der Proteine automatisch erzeugt werden.

Gemäß Figur 3 kann auf der Basis der von der Speichereinrichtung 5 abgerufenen Daten betreffend potentielle Wechselwirkungen zwischen Proteinen auch eine Visualisierung automatisch erzeugt werden, in welcher von den verschiedenen Daseinsformen A1, A2, A3, B1 und B4 jeweils eigene Unterknoten gebildet sind. Mit Hilfe von Kanten 30, 31 wird in Figur 3 die potentielle Wechselwirkung zwischen der Daseinsform A1 und dem Wechselwirkungspartner (Knoten WWP1) sowie zwischen der Daseinsform B1 und dem Wechselwirkungspartner (Knoten WWP1) dargestellt. Auf diese Weise ist es möglich, die in Figur 2 ausgegebenen Informationen über mögliche Wechselwirkungen zwischen verschiedenen Proteinen, die teilweise einer bestimmten Daseinsform angehören, weiter zu spezifizieren, um es dem Benutzer zu erleichtern, aus der Vielzahl der in der Speichereinrichtung 5 gespeicherten, elektronischen Daten die für den Anwendungsfall relevanten Informationen herauszufiltern. Der Benutzer kann mit Hilfe einer Eingabe über die Eingabeeinrichtung 2 die Knoten- und Kantenbildung interaktiv beeinflussen, insbesondere die in Figur 2 schematisch dargestellte Ausgabe in die in Figur 3 schematisch dargestellte Ausgabe überführen, und umgekehrt.

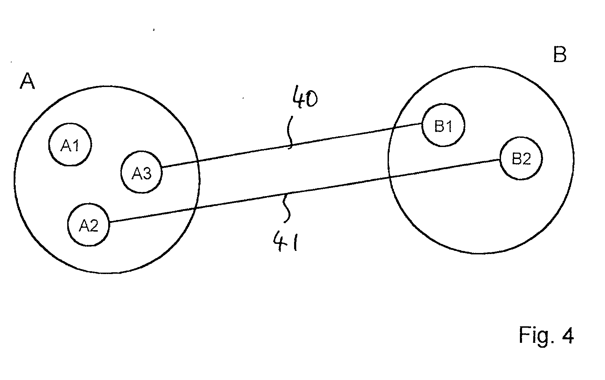

Gemäß Figur 4 ist es mit Hilfe der Anordnung nach Figur 1 auch möglich, Kanten 40 bzw. 41 zu erzeugen, die eine mögliche Wechselwirkung zwischen den Daseinsformen A3 und B1 bzw. zwischen den Daseinsformen A2 und B2 repräsentiert.

Mit Hilfe entsprechender Eingaben über die Eingabeeinrichtung 2 kann der Benutzer zwischen den verschiedenen Ausführungsformen nach den Figuren 2, 3 und 4 umschalten und auf diese Weise interaktiv den Informationsgehalt der in der Datenspeichereinrichtung 5 vorhandenen elektronischen Daten für die gegebene Problemstellung nutzen.

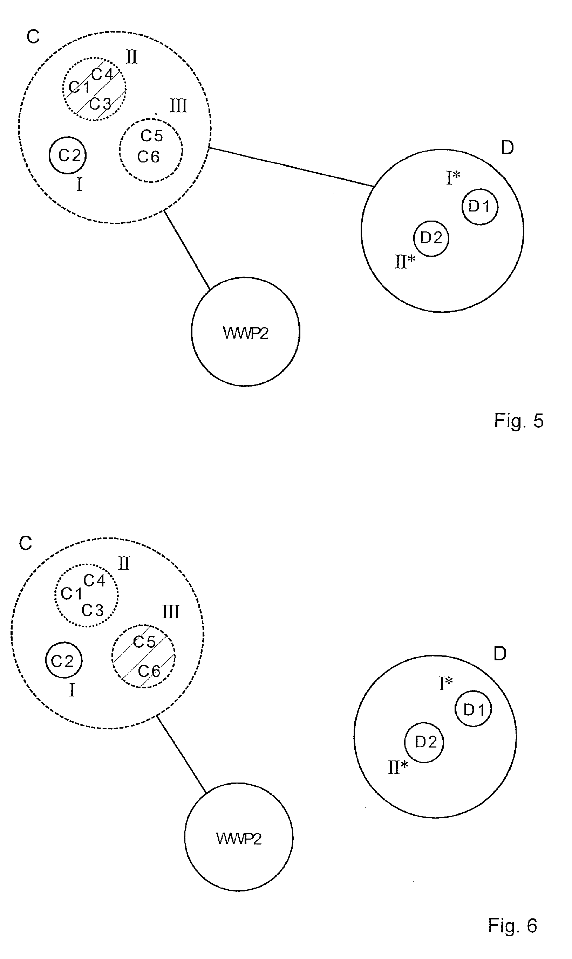

Figur 5 zeigt eine Anordnung von weiteren Knoten C, D und WWP2 und Kanten 50, 51. Hierbei wurden auf der Basis der von der Speichereinrichtung 5 abgerufenen Daten und der automatischen Berücksichtigung von Benutzereingaben, die der Benutzer unter Nutzung geeigneter Eingaben über ein Eingabemenü eingibt, Unterknoten I, II, III bzw. I*, II* gebildet, wobei die Unterknoten I, II, III bzw. I*, II* jeweils Daseinsformen eines Proteins C bzw. D mit mindestens einer gemeinsamen Eigenschaft umfassen. Die gemeinsame Eigenschaft betrifft beispielsweise die Tatsache, daß die zum Unterknoten I gehörenden Daseinsformen nur bei Menschen, die eine bestimmte Krankheit haben, und die zum Unterknoten II gehörenden Daseinsformen nur bei Menschen auftreten, die gesund sind. Grundsätzlich können vorzugsweise beliebige biologische Kriterien herangezogen werden, um die Unterknoten zu bilden. Der Benutzer kann dann mit Hilfe einer Eingabe über die Eingabeeinrichtung 2 einen der Unterknoten I, II bzw. III auswählen, um automatisch (eine) Kante(n) zu dem oder den Knoten (WWP2, D) der potentiellen Wechselwirkungspartner zu erzeugen und auszugeben, die mit den Daseinsformen in dem ausgewählten Unterknoten I, II bzw. III wechselwirken. Wenn der Benutzer den Unterknoten II auswählt, so können beispielsweise die Kanten 50 und 51 erzeugt werden, d.h. eine oder mehrere Gensequenz-Daseinsformen des Proteins C, die zum Unterknoten II gehören, wechselwirken potentiell mit dem Protein WWP2 (vgl. Figur 5). Wenn der Benutzer jedoch den Unterknoten III auswählt, so wird die Kante 52 erzeugt, d.h. eine oder mehrere Gensequenz-Daseinsformen des Proteins C, die zum Unterknoten III gehören, wechselwirken potentiell mit dem Protein WWP2 (vgl. Figur 6). Die Auswahl des Unterknotens II bzw. III ist in den Figuren 5 bzw. 6 mittels einer jeweiligen Schraffur schematisch dargestellt. Auf diese Weise kann der Benutzer die umfangreichen elektronischen Daten aus der Datenspeichereinrichtung 5 für den Anwendungsfall spezifiziert visualisieren.

In den Figuren 2 bis 6 wurden als grafische Ausgabeelemente für die Knoten beispielhaft Kreise gewählt. Es können jedoch beliebige grafische Elemente vorgesehen sein, die eine grafische Ausgabe der Knoten ermöglichen, z.B. Vielecke.

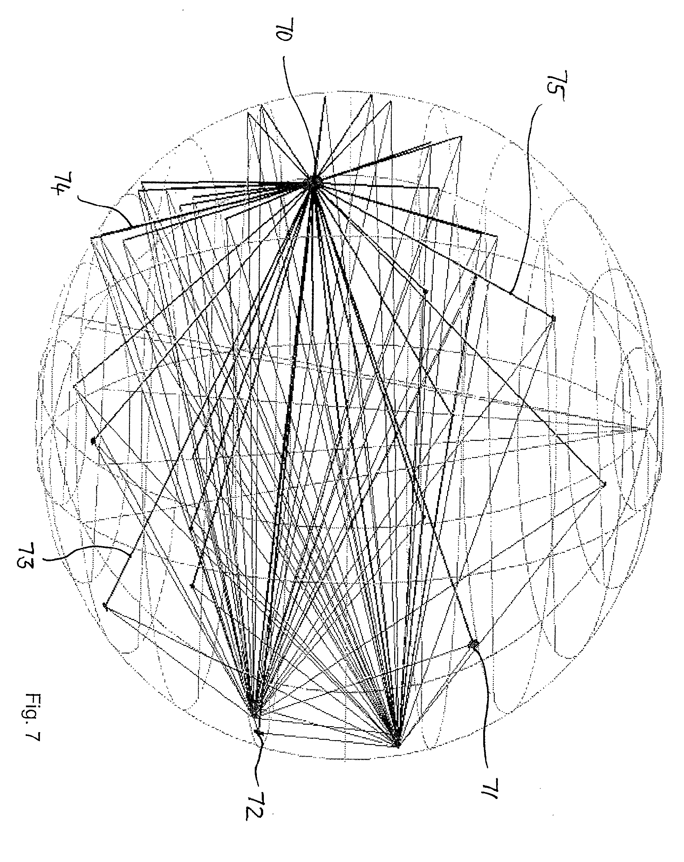

Eine für den Benutzer besonders übersichtliche Visualisierung der möglichen Protein-Protein-Wechselwirkungen auf der Basis der automatisch erzeugten Kanten und Knoten wird dadurch erreicht, daß die Knoten gemäß Figur 7 auf einer Kugeloberfläche und die Kanten als Verbindungen zwischen den Knoten auf der Kugeloberfläche grafisch dargestellt werden. IN Figur 7 sind beispielhaft Knoten 70, 71, 72 und Kanten 73, 74, 75 bezeichnet. Mittels geeigneter Eingaben über die Eingabeeinrichtung 2, die beispielsweise als eine Mauseinrichtung oder eine Tastatur ausgebildet sein kann, kann der Benutzer einzelne oder mehrere Knoten zur besseren Übersichtlichkeit in die Mitte der Darstellung holen. Die hyperbolische Darstellung erlaubt dann eine hohe Auflösung des Netzwerks der Kanten zwischen den Knoten in diesem Bereich, während eine Raumkompression zum Sphärenrand immer mehr zunimmt. Auf diese Art und Weise ist eine übersichtliche und benutzerfreundliche Darstellung auch sehr großer Netzwerke ermöglicht.

Die in der vorstehenden Beschreibung, der Zeichnung und den Ansprüchen offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verfahren zum automatischen Verarbeiten und Ausgeben von elektronischen Daten aus einer Datenspeichereinrichtung, wobei die elektronischen Daten eine erste Folge elektronischer Abfragedaten, zumindest eine zweite Folge elektronischer Abfragedaten und elektronische Testdaten umfassen, wobei die erste Folge elektronischer Abfragedaten eine erste Folge von Objekten elektronisch repräsentiert, die wenigstens eine gemeinsame Eigenschaft aufweisen, wobei die zumindest eine zweite Folge elektronischer Abfragedaten eine zweite Folge von Objekten elektronisch repräsentiert, die wenigstens eine gemeinsame Eigenschaft aufweisen, und wobei die elektronischen Testdaten wenigstens ein Testobjekt elektronisch repräsentieren, das Verfahren die folgenden Schritte aufweisend:
- automatisches Erfassen einer Schlüsselwort-Benutzereingabe;
- Abrufen der ersten Folge elektronischer Abfragedaten über eine Abrufeinrichtung einer elektronischen Verarbeitungseinrichtung, elektronisches Ausbilden eines ersten Knotens, welcher die erste Folge elektronischer Abfragedaten umfaßt, und automatisches Erzeugen erster elektronischer Signale in der elektronischen Verarbeitungseinrichtung zum Ausgeben einer grafischen Repräsentation des ersten Knotens;
- Abrufen der zumindest einen zweiten Folge elektronischer Abfragedaten über die Abrufeinrichtung der elektronischen Verarbeitungseinrichtung, elektronisches Ausbilden zumindest eines zweiten Knotens, welcher die zumindest eine zweite Folge elektronischer Abfragedaten umfaßt, und automatisches Erzeugen zweiter elektronischer Signale zum Ausgeben einer grafischen Repräsentation des zweiten Knotens;
- Abrufen wenigstens eines Teils der elektronischen Testdaten über die Abrufeinrichtung der elektronischen Verarbeitungseinrichtung, elektronisches Ausbilden wenigstens eines weiteren Knotens, welcher den wenigstens einen Teil der elektronischen Testdaten umfaßt, und automatisches Erzeugen weiterer elektronischer Signale zum Ausgeben einer grafischen Repräsentation des wenigstens einen weiteren Knotens;
- elektronisches Ausbilden von Kanten zwischen dem ersten Knoten und/oder dem zweiten Knoten und/oder dem weiteren Knoten in Abhängigkeit von der erfaßten Schlüsselwort-Benutzereingabe und automatisches Erzeugen anderer elektronischer Signale zum Ausgeben einer grafischen Repräsentation der Kanten mit Hilfe der elektronischen Verarbeitungseinrichtung; und
- Übermitteln der ersten elektronischen Signale, der zweiten elektronischen Signale, der weiteren elektronischen Signale und der anderen elektronischen Signale sowie jeweils zugehöriger Informationen zum Unterscheiden des ersten Knotens, des zumindest einen zweiten Knotens, des weiteren Knotens bzw. der Kanten von der elektronischen Verarbeitungseinrichtung an wenigstens eine Ausgabeeinrichtung zum Ausgeben über die wenigstens eine Ausgabeeinrichtung.

2. Verfahren nach Anspruch 1, **gekennzeichnet durch** die weiteren Verfahrensschritte:
- automatisches Erfassen einer weiteren Schlüsselwort-Benutzereingabe;
- elektronisches Ausbilden von ersten Unterknoten des ersten Knotens und automatisches Erzeugen elektronischer Signale in der elektronischen Verarbeitungseinrichtung zum Ausgeben einer grafischen Repräsentation der ersten Unterknoten;
- elektronisches Ausbilden von zweiten Unterknoten des zumindest einen zweiten Knotens und automatisches Erzeugen elektronischer Signale in der elektronischen Verarbeitungseinrichtung zum Ausgeben einer grafischen Repräsentation der zweiten Unterknoten;
- elektronisches Ausbilden von weiteren Kanten zwischen wenigstens einem Teil der ersten Unterknoten und dem weiteren Knoten und/oder zwischen wenigstens einem Teil der zweiten Unterknoten und dem weiteren Knoten in Abhängigkeit von der weiteren erfaßten Schlüsselwort-Benutzereingabe und automatisches Erzeugen elektronischer Signale mit Hilfe der elektronischen Verarbeitungseinrichtung zum Ausgeben einer grafischen Repräsentation der weiteren Kanten; und
- Übermitteln der erzeugten elektronischen Signale und jeweils zugehöriger Informationen zum Unterscheiden des wenigstens einen Teils der ersten Unterknoten, des wenigstens einen Teils der zweiten Unterknoten, der weiteren Knoten bzw. der weiteren Kanten von der elektronischen Verarbeitungseinrichtung an die wenigstens eine Ausgabeeinrichtung zum Ausgeben über die wenigstens eine Ausgabeeinrichtung.

3. Verfahren nach Anspruch 2, **gekennzeichnet durch** die weiteren Verfahrensschritte:
- automatisches Erfassen einer anderen Schlüsselwort-Benutzereingabe;
- elektronisches Ausbilden von anderen Kanten zwischen wenigstens einem Teil der ersten Unterknoten und wenigstens einem Teil der zweiten Unterknoten in Abhängigkeit von der anderen erfaßten Schlüsselwort-Benutzereingabe und automatisches Erzeugen elektronischer Signale mit Hilfe der elektronischen Verarbeitungseinrichtung zum Ausgeben einer grafischen Repräsentation der anderen Kanten; und
- Übermitteln der erzeugten elektronischen Signale und jeweils zugehöriger Informationen zum Unterscheiden des wenigstens einen Teils der ersten Unterknoten, des wenigstens einen Teils der zweiten Unterknoten bzw. der anderen Kanten von der elektronischen Verarbeitungseinrichtung an die wenigstens eine Ausgabeeinrichtung zum Ausgeben über die wenigstens eine Ausgabeeinrichtung.

4. Verfahren nach einem der vorangehenden Ansprüche, **dadurch gekennzeichnet, daß** das erste elektronische Signal, das zweite elektronische Signal, das weitere elektronische Signal und das andere elektronische Signal so ausgebildet sind, daß der erste Knoten, der zumindest eine zweite Knoten und der weitere Knoten als grafische Elemente auf einer Kugeloberfläche und die Kanten als grafischen Verbindungen zwischen den grafischen Elementen ausgegeben werden.

5. Verfahren nach Anspruch 2 oder 3, **dadurch gekennzeichnet, daß** die elektronischen Signale und das andere elektronische Signal so ausgebildet sind, daß der wenigstens eine Teil der ersten Unterknoten, der wenigstens einen Teil der zweiten Unterknoten und der weitere Knoten als weitere grafische Elemente auf einer Kugeloberfläche und die weiteren und/oder die anderen Kanten als weitere bzw. andere grafische Verbindungen zwischen den weiteren grafischen Elementen ausgegeben werden.

6. Vorrichtung zum automatischen Verarbeiten und Ausgeben von elektronischen Daten aus einer Datenspeichereinrichtung, wobei die elektronischen Daten eine erste Folge elektronischer Abfragedaten, zumindest eine zweite Folge elektronischer Abfragedaten und elektronische Testdaten umfassen, wobei die erste Folge elektronischer Abfragedaten eine erste Folge von Objekten elektronisch repräsentiert, die wenigstens eine gemeinsame Eigenschaft aufweisen, wobei die zumindest eine zweite Folge elektronischer Abfragedaten eine zweite Folge von Objekten elektronisch repräsentiert, die wenigstens eine gemeinsame Eigenschaft aufweisen, und wobei die elektronischen Testdaten wenigstens ein Testobjekt elektronisch repräsentieren, die Vorrichtung aufweisend:
- eine Erfassungseinrichtung zum automatischen Erfassen einer Schlüsselwort-Benutzereingabe;
- eine Abrufeinrichtung zum Abrufen der elektronischen Daten von der Datenspeichereinrichtung;
- eine Prozessoreinrichtung mit einer Erzeugungseinrichtung zum
- automatischen Erzeugen eines ersten Knotens, welcher die erste Folge elektronischer Abfragedaten umfaßt, und erster elektronischer Signale zum Ausgeben einer grafischen Repräsentation des ersten Knotens;
- automatischen Erzeugen zumindest eines zweiten Knotens, welcher die zumindest eine zweite Folge elektronischer Abfragedaten umfaßt, und zweiter elektronischer Signale zum Ausgeben einer grafischen Repräsentation des zweiten Knotens;
- automatischen Erzeugen wenigstens eines weiteren Knotens, welcher den wenigstens einen Teil der elektronischen Testdaten umfaßt, und weiterer elektronischer Signale zum Ausgeben einer grafischen Repräsentation des wenigstens einen weiteren Knotens;
- automatischen Ausbilden von Kanten zwischen dem ersten Knoten und/oder dem zweiten Knoten und/oder dem weiteren Knoten in Abhängigkeit von der erfaßten Schlüsselwort-Benutzereingabe und Erzeugen anderer elektronischer Signale zum Ausgeben einer grafischen Repräsentation der Kanten; und
- eine Übermittlungseinrichtung zum Übermitteln der elektronischen Signale an wenigstens eine Ausgabeeinrichtung.

7. Computerprogrammprodukt, welches in einen internen Speicher einer Computereinrichtung ladbar ist und welches Computerprogrammabschnitte zum Ausführen der Verfahrensschritte nach einem der Ansprüche 1 bis 5 beim Abarbeiten des Computerprogrammprodukts in der Computereinrichtung umfaßt.

8. Computerprogrammprodukt nach Anspruch 7, wobei wenigstens die Computerprogrammabschnitte auf einem von der Computereinrichtung lesbaren Speichermedium gespeichert sind.

9. Elektronische Datenstruktur, die mit Hilfe eines Verfahrens nach einem der Ansprüche 1 bis 5 gebildet wird und auf einem physikalischen Speicher gespeichert ist.
